# EUROPÄISCHE PATENTSCHRIFT

(11) **EP 2 588 221 B1**
(45) Veröffentlichungstag und Bekanntmachung des Hinweises auf die Patenterteilung: **23.04.2014**
(21) Anmeldenummer: 11725860.8
(22) Anmeldetag: 07.06.2011
(51) Int. Cl.: A61J 1/20, A61M 5/178, B01F 15/00, A61M 5/31, A61M 5/315, B01F 11/00, B01F 13/00

(54) **KOMBINIERTE MISCH- UND AUSTRAGVORRICHTUNG**
COMBINED MIXING AND DISCHARGING DEVICE
DISPOSITIF COMBINÉ DE MÉLANGE ET DE DÉVERSEMENT

(30) Priorität: 01.07.2010 CH 10702010
(43) Veröffentlichungstag der Anmeldung: 08.05.2013
(73) Patentinhaber: Medmix Systems AG, 6343 Rotkreuz (CH)
(72) Erfinder: GRETER, Andy, CH-6340 Baar (CH)
(74) Vertreter: Detken, Andreas
(86) Internationale Anmeldenummer: PCT/CH2011/000135
(87) Internationale Veröffentlichungsnummer: WO 2012/000122

(56) Entgegenhaltungen:
- EP-A1- 0 974 373
- WO-A1-03/084445
- WO-A1-2009/105905
- CH-A- 541 481
- US-A- 3 195 778

## Beschreibung

### TECHNISCHES GEBIET

Die vorliegende Erfindung betrifft eine Austragvorrichtung zum Mischen und Austragen eines Produktes. Die Austragvorrichtung weist ein Gehäuse auf, einen darin verschiebbaren Kolben sowie ein Vorschubelement, an welchem ein Mischelement ausgebildet ist. Eine derartige Austragvorrichtung kann zum Beispiel dazu dienen, eine erste Komponente zu lagern, eine zweite Komponente unmittelbar vor der Applikation aufzunehmen, diese mit der ersten Komponente zu vermischen und das vermischte Produkte anschliessend auszutragen bzw. zu applizieren.

### STAND DER TECHNIK

Austragvorrichtungen in Form von Spritzen sind seit langem allgemein bekannt. Eine handelsübliche Spritze weist ein Gehäuse mit einer distalen Austrittsöffnung und ein Vorschubelement auf, das von einem proximalen Ende her in das Gehäuse einschiebbar ist. Das Vorschubelement weist eine Kolbenstange mit einem distal daran angebrachten Kolben auf, der dichtend im Innenraum des Spritzenkörpers verschiebbar ist. Um ein Fluid in der Spritze aufzunehmen, wird durch Zurückziehen des Vorschubelementes in proximaler Richtung ein Unterdruck im Gehäuse gebildet, wodurch das Fluid durch die distale Austrittsöffnung eingesaugt wird. Beim anschliessenden Verschieben des Vorschubelementes in die distale Richtung wird das Fluid durch die Austrittsöffnung wieder ausgestossen.

Einige fluide Produkte, die mittels einer solchen Austragvorrichtung appliziert werden sollen, müssen unmittelbar vor der Anwendung verrührt oder vermischt werden. Das fluide Produkt kann dabei bereits in vollständiger Zusammensetzung in der Austragvorrichtung aufbewahrt sein, oder es ist möglich, dass mehrere Komponenten des fluiden Produkts innerhalb der Austragvorrichtung zusammengeführt und vermischt werden müssen. Es sind insbesondere Anwendungen bekannt, bei denen in der Austragvorrichtung eine pulverförmige Substanz gelagert ist, zu welcher unmittelbar vor der Anwendung eine oder mehrere Flüssigkeiten hinzugeführt und mit ihr vermischt werden, z.B. zur Zubereitung eines Knochenzements, aber auch in pharmazeutischen Anwendungen. Um ein ausreichendes Mischen insbesondere auch bei zähflüssigen Fluiden innerhalb der Austragvorrichtung zu ermöglichen, bestehen Austragvorrichtungen, die eine Mischeinrichtung umfassen.

Eine derartige Austragvorrichtung mit einer Mischeinrichtung ist beispielsweise im Dokument US 7,736,049 oder im Dokument WO 2007/003063 offenbart. Zwei spritzenartige Behälter sind parallel nebeneinander angeordnet und an ihren Auslässen über eine Ventileinrichtung miteinander verbunden. Der erste, grössere Behälter enthält z.B. ein Pulver, während der zweite, kleinere Behälter eine Flüssigkeit aufnimmt. Im ersten Behälter ist ein verschiebbarer Kolben vorhanden, durch den hindurch eine Mischstange mit einem daran angebrachten Mischelement geführt ist. Im zweiten Behälter ist ebenfalls ein Kolben vorhanden, der mit einer Kolbenstange versehen ist. Um die Flüssigkeit vom ersten in den zweiten Behälter zu transferieren, wird der Kolben des zweiten Behälters mittels der Kolbenstange in Richtung des Auslasses vorgeschoben, so dass die im zweiten Behälter aufgenommene Flüssigkeit in den ersten Behälter gepresst wird. Anschliessend wird das resultierende Gemisch aus Pulver und Flüssigkeit durch Hin- und Herbewegen der Mischstange durchmischt, bevor das Gemisch durch Vorschieben des Kolbens des ersten Behälters ausgetragen wird.

Wenn bei einer solchen Vorrichtung die Flüssigkeit in den ersten Behälter gepresst wird, verdrängt und komprimiert sie die im ersten Behälter vorhandene Luft. Dadurch kann im ersten Behälter ein gefährlicher Überdruck entstehen. Daher ist es wünschenswert, den ersten Behälter so auszugestalten, dass im Bereich des Kolbens nötigenfalls Luft entweichen kann.

Wenn der erste Behälter allerdings so ausgestaltet wird, dass der Bereich des Kolbens für Luft durchlässig ist, ist es nicht möglich, die Flüssigkeit dadurch in den ersten Behälter aufzusaugen, dass der Kolben des ersten Behälters zurückgezogen wird, da kein Vakuum im ersten Behälter erzeugt werden kann. Es ist also nicht möglich, mit ein und derselben Vorrichtung wahlweise die Flüssigkeit durch Einschieben des Kolbens in den zweiten Behälter oder durch Zurückziehen des ersten Kolbens zu befüllen, sondern der Benutzer ist aufgrund der Konstruktion der Austragvorrichtung auf eine dieser beiden Möglichkeiten festgelegt. Zudem ist es nachteilig, wenn Luft durch den Bereich des Kolbens in den ersten Behälter eindringen kann, weil dadurch während der Lagerung unter Umständen die chemischen Eigenschaften des im ersten Behälter aufgenommenen Produkts verändert werden.

Eine weitere Austragvorrichtung mit einer Mischeinrichtung ist im Dokument WO 2009/105905 offenbart. Die Austragvorrichtung weist einen Behälter mit einem darin verschiebbaren Kolben auf, durch welchen hindurch eine Mischstange geführt ist. Am distalen Ende der Mischstange ist ein Mischelement angeordnet. Die Mischstange ist gegenüber dem Kolben verschiebbar, kann jedoch mittels eines Sicherungselementes am Kolben fixiert werden. Die zu applizierenden Substanzen können somit, wenn die Mischstange am Kolben fixiert wurde, in den Behälter aufgezogen und aus diesem ausgestossen werden. Im nicht fixierten Zustand, beim Vermischen der Substanzen, bleibt der Kolben unbeweglich relativ zum Behälter, während die Mischstange mit dem Mischelement in Längsrichtung vor- und zurückbewegt wird.

Bei einer derartigen Ausgestaltung ist es zwar ohne Weiteres möglich, eine Flüssigkeit durch Zurückziehen des Kolbens in den Behälter aufzusaugen. Falls der Benutzer den Behälter jedoch dadurch mit Flüssigkeit befüllen möchte, dass er die Flüssigkeit mittels einer separaten Spritze in den Behälter einspritzt, kann im Behälter wiederum ein gefährlicher Überdruck entstehen. Auch beim Vermischen der im Gehäuse aufgenommenen Substanzen mittels der Mischeinrichtung kann bei einer solchen Vorrichtung ein Unter- oder Überdruck entstehen. Ein verbleibender Über- bzw. Unterdruck im Behälter kann beim anschliessenden Öffnen der Austrittsöffnung dazu führen, dass ein Teil des Produktes aus dem Behälter herausspritzt, bzw. dass Luft hineingesogen wird. Dies ist insbesondere bei giftigen, stark ätzenden oder auch bei exakt zu dosierenden Produkten problematisch.

Nach eine weitere Austragvorrichtung ist aus der WO 03/084445 bekannt.

### DARSTELLUNG DER ERFINDUNG

Es ist eine erste Aufgabe der vorliegenden Erfindung, eine Austragvorrichtung mit einer Mischeinrichtung anzugeben, die es ermöglicht, eine Flüssigkeit wahlweise durch die Anwendung von Unterdruck aufzuziehen oder durch Einspritzen von aussen in die Austragvorrichtung zu überführen, ohne dass dabei ein gefährlicher Überdruck entsteht. Es ist eine alternative zweite Aufgabe der vorliegenden Erfindung, eine Austragvorrichtung mit einer Mischeinrichtung anzugeben, die es ermöglicht, eine Flüssigkeit durch Einspritzen von aussen in die Austragvorrichtung zu überführen, ohne dass dabei ein gefährlicher Überdruck entsteht, die aber dennoch sicherstellt, dass während der Lagerung keine Luft in die Austragvorrichtung gelangen kann. Es ist eine alternative dritte Aufgabe der vorliegenden Erfindung, eine Austragvorrichtung mit einer Mischeinrichtung anzugeben, die es ermöglicht, eine Flüssigkeit durch die Anwendung von Unterdruck aufzuziehen, bei welcher aber das Entstehen eines Über- oder Unterdruckes während des Mischvorgangs vermieden wird. Jede dieser Aufgaben wird durch eine Vorrichtung mit den Merkmalen des Anspruchs 1 gelöst. Weitere Ausführungsformen sind in den abhängigen Ansprüchen angegeben.

Die vorliegende Erfindung stellt also eine Austragvorrichtung zum Mischen und Austragen eines Produktes zu Verfügung, aufweisend
ein Gehäuse, welches ein Reservoir für das Produkt begrenzt, mit einer umlaufenden Seitenwand, einem offenen proximalen Gehäuseende und einem distalen Gehäuseende mit einer Austrittsöffnung;
ein Vorschubelement, welches vom proximalen Gehäuseende her in das Gehäuse hineinragt und entlang einer Längsrichtung relativ zum Gehäuse verschiebbar ist, wobei das Vorschubelement einen distalen Bereich mit einem Mischelement zum Vermischen des Produktes aufweist; und
einen Kolben, welcher proximal in Bezug auf das Mischelement angeordnet ist, und welcher relativ zum Gehäuse in Längsrichtung verschiebbar ist, um das Produkt durch die Austrittsöffnung hindurch aus dem Reservoir auszustossen.

Der Kolben weist einen ungekoppelten Zustand auf, in welchem das Vorschubelement relativ zum Kolben in Längsrichtung verschiebbar ist, und weist einen mit dem Vorschubelement gekoppelten Zustand auf, in welchem der Kolben mittels des Vorschubelements in Längsrichtung relativ zum Gehäuse verschiebbar ist.

Der Kolben dichtet das Reservoir im gekoppelten Zustand zusammen mit dem Vorschubelement in proximaler Richtung luftdicht nach aussen hin ab. Im ungekoppelten Zustand geben der Kolben und/oder das Vorschubelement mindestens eine Entlüftungsöffnung in proximaler Richtung nach aussen hin frei.

Indem der Kolben im gekoppelten Zustand mittels des Vorschubelements zurückgezogen wird, wird es möglich, eine Flüssigkeit ins Reservoir aufzusaugen. Wenn dagegen eine Flüssigkeit mittels einer Spritze in die Austragvorrichtung eingespritzt werden soll, kann der Kolben in den ungekoppelten Zustand gebracht werden. Im Gehäuse vorhandene Luft kann dann durch die Entlüftungsöffnung nach aussen hin entweichen, wodurch die Entstehung eines Überdrucks vermieden wird.

Ausserdem kann im ungekoppelten Zustand ein Über- bzw. Unterdruck, welcher möglicherweise beim Vermischen von zwei oder mehr im Reservoir aufgenommenen Komponenten entsteht, nach aussen hin ausgeglichen werden. Ein derartiger Über- oder Unterdruck kann insbesondere dann entstehen, wenn beim Vermischen der Komponenten das Vorschubelement, welches eine Kolbenstange mit daran angebrachtem Mischelement aufweisen kann, relativ zum Gehäuse vor- oder zurückgeschoben wird. Beim Vorschieben des Vorschubelementes in das Gehäuse hinein ragt dieses immer weiter in das Reservoir hinein und beansprucht aufgrund seines Verdrängervolumens zunehmend mehr Platz, wodurch im Reservoir ein Überdruck entstehen kann. Beim Zurückziehen des Vorschubelements relativ zum Gehäuse kann entsprechend ein Unterdruck entstehen. Ein Über- oder Unterdruck kann auch durch chemische Reaktionen während des Vermischens entstehen, z.B. Polymerisationsreaktionen unter Abspaltung eines Gases. Indem die Austragvorrichtung mindestens eine Entlüftungsöffnung aufweist, kann dieser Über- bzw. Unterdruck nach aussen hin ausgeglichen werden.

Im gekoppelten Zustand ist das Reservoir für die Lagerung, das Aufziehen und das Ausstossen des Produktes dennoch luftdicht in die proximale Richtung verschlossen.

Im Folgenden werden Richtungsangaben wie folgt verwendet. Die Längsrichtung bezeichnet die Verschiebungsrichtung des Vorschubelementes. Die distale Richtung ist jeweils diejenige Richtung, in welche der Kolben zum Austragen eines in der Austragvorrichtung aufgenommenen Produktes zu einer Austrittsöffnung hin vorgeschoben wird. Die proximale Richtung bezeichnet die dazu entgegengesetzte Richtung.

Bei dem im Reservoir aufgenommenen Produkt kann es sich beispielsweise um mehrere Komponenten eines zu mischenden Medikamentes oder um mehrere Komponenten eines medizinischen oder nicht-medizinischen Klebstoffes oder eines Knochenzements handeln. Im Lagerungszustand ist dabei vorteilhaft nur eine dieser Komponenten in der Austragvorrichtung aufgenommen. Weitere Komponenten können dann beispielsweise unmittelbar vor der Anwendung oder Verabreichung des Produktes zum Beispiel von einem Vial oder einer handelsüblichen Spritze in das Reservoir übertragen werden.

Im gekoppelten Zustand des Kolbens ist die Austragvorrichtung wie eine handelsübliche Spritze verwendbar, das heisst ein fluides Produkt ist mit Hilfe des Vorschubelementes in das Reservoir aufziehbar bzw. aus diesem ausstossbar. Da das Reservoir dann in proximaler Richtung luftdicht abgedichtet ist, entsteht beim Herausziehen des Vorschubelementes aus dem Gehäuse heraus im Reservoir ein Unterdruck und beim Vorschieben des Vorschubelementes in das Gehäuse hinein ein Überdruck, wobei der Über- bzw. Unterdruck dann erwünscht ist, um das Produkt auszustossen bzw. aufzuziehen. Vorzugsweise ist der Kolben dabei im gekoppelten Zustand derart mit dem Vorschubelement verbunden, dass er in Längsrichtung relativ zum Vorschubelement unbeweglich ist.

Bevorzugt erstreckt sich das Vorschubelement durch den Kolben hindurch. Im gekoppelten Zustand liegt der Kolben dann sowohl am Vorschubelement als auch am Gehäuse luftdicht an, um das Reservoir in die proximale Richtung vollständig abzudichten. Im ungekoppelten Zustand kann dann beispielsweise ein Luftdurchgang zwischen Kolben und Vorschubelement und/oder zwischen Kolben und Gehäuse vorhanden sein.

Bevorzugt ist die zumindest eine Entlüftungsöffnung am Kolben ausgebildet, wobei das

Vorschubelement die Entlüftungsöffnung im gekoppelten Zustand luftdicht verschliesst. In einer bevorzugten Ausführungsform ist die zumindest eine Entlüftungsöffnung im gekoppelten Zustand dabei durch das Mischelement verschlossen. Das Mischelement kann dann in einem der Entlüftungsöffnung gegenüberliegenden Bereich ein entsprechend ausgebildetes Verschliesselement aufweisen.

Vorteilhaft ist die zumindest eine Entlüftungsöffnung als eine radiale Vertiefung ausgebildet. Das heisst, die Entlüftungsöffnung ist dann nicht vollständig vom Material des Kolbens umgeben. Die radiale Vertiefung kann dabei insbesondere auf einer radialen Aussen- oder Innenseite des Kolbens ausgebildet sein.

Bevorzugt ist die Entlüftungsöffnung jedoch an einer radialen Innenseite des Kolbens ausgebildet. Vorteilhaft weist dann die radiale Innenseite des Kolbens im Bereich der Entlüftungsöffnung einen radial umlaufenden Kontaktbereich auf, welcher sich in distaler Richtung radial nach aussen hin aufweitet. Der Kontaktbereich kann dabei eine Flächennormale aufweisen, die in Bezug auf die Längsrichtung geneigt ist, insbesondere in einem Winkel von ca. 45°. Falls der Kolben einen derartigen Kontaktbereich aufweist, ist am Vorschubelement vorteilhaft ein radial umlaufender Absatz mit einer Aussenkante vorhanden, welche im gekoppelten Zustand umlaufend luftdicht am Kontaktbereich anliegt.

Vorteilhaft dichten der Kolben und das Vorschubelement das Reservoir sowohl im gekoppelten Zustand als auch im ungekoppelten Zustand derart ab, dass das Reservoir in die proximale Richtung in Bezug auf pulver- und pastenförmige Substanzen nach aussen hin undurchlässig ist. Ein Austreten des im Reservoir gelagerten Produktes im ungekoppelten Zustand und insbesondere während dem Mischvorgang wird dadurch verhindert.

Bevorzugt weist der Kolben eine Trägerhülse sowie ein an der Trägerhülse angebrachtes Dichtelement auf, welches die Trägerhülse umgibt. Die Trägerhülse dient zum Halten des Dichtelementes. Das Dichtelement ist dabei vorteilhaft aus einem flexiblen und elastischen Material hergestellt, während das Halteelement im Vergleich dazu starr ausgebildet ist. Vorteilhaft ist dabei die Entlüftungsöffnung am Dichtelement ausgebildet.

Im ungekoppelten Zustand kann ein Verschieben des Kolbens relativ zum Gehäuse in die proximale Richtung dazu führen, dass Luft von aussen her in das Reservoir hinein gesogen wird. Dies ist vorteilhaft dadurch verhindert, dass der Kolben nur dann vom Vorschubelement entkoppelbar ist, wenn der Kolben bis zu einem proximalen Anschlag aus dem Gehäuse herausgezogen ist. Dazu kann die Austragvorrichtung beispielsweise ein Sicherungselement aufweisen, mittels welchem der Kolben mit dem Vorschubelement koppelbar ist. Das Sicherungselement kann dann jedoch zum Beispiel so ausgebildet sein, dass es ein Entkoppeln ausschliesslich dann erlaubt, wenn der Kolben bis zu einem proximalen Anschlag aus dem Gehäuse herausgezogen ist.

Das Sicherungselement ist bevorzugt derart in Längsrichtung verschiebefest an das Vorschubelement anbringbar, dass der Kolben in Längsrichtung zwischen dem Sicherungselement und dem Vorschubelement fixiert ist und dadurch mit dem Vorschubelement gekoppelt ist. Das Sicherungselement kann dabei insbesondere entfernbar vom Vorschubelement sein, um den Kolben vom Vorschubelement zu entkoppeln. Vorteilhaft ist das Sicherungselement jedoch nur dann vom Vorschubelement entfernbar, wenn der Kolben bis zu einem proximalen Anschlag aus dem Gehäuse herausgezogen ist. Dies kann insbesondere zum Beispiel dadurch realisiert sein, dass das Sicherungselement eine langgestreckte Form aufweist und zusammen mit dem Vorschubelement in das Reservoir einschiebbar ist. Ein Entfernen des Sicherungselementes vom Vorschubelement ist dann aufgrund der radialen Begrenzung durch das Gehäuse vorteilhaft nur dann möglich, wenn das Sicherungselement zusammen mit dem Vorschubelement zu einem Grossteil aus dem Gehäuse herausgezogen ist. Das Sicherungselement kann auch, wie zum Beispiel in der WO 2009/105905 beschrieben ist, schwenkbar mit einem proximalen Bereich des Vorschubelementes verbunden sein. Ein Verschwenken des Sicherungselementes gegenüber dem Vorschubelement, um den Kolben mit dem Vorschubelement zu koppeln bzw. zu entkoppeln, ist dann nur möglich, wenn das Sicherungselement aus dem Gehäuse herausgezogen ist und das Vorschubelement und der Kolben einen proximalen Anschlag mit dem Gehäuse bilden.

Vorteilhaft bildet der Kolben im gekoppelten Zustand einen distalen Anschlag am Mischelement.

Das Sicherungselement bildet im gekoppelten Zustand in proximaler Richtung bevorzugt einen Anschlag mit einem Element des Vorschubelementes, welches im Bereich des proximalen Endes des Vorschubelements angeordnet ist. Dabei bildet das Sicherungselement im gekoppelten Zustand vorteilhaft in proximaler Richtung einen Anschlag mit einem am Vorschubelement angebrachten Betätigungsgriff und in distaler Richtung einen Anschlag mit dem Kolben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine zentrale Schnittansicht durch eine erfindungsgemässe Austragvorrichtung gemäss einer ersten Ausführungsform, mit daran angeschlossenem Vial, beim Aufziehen einer Flüssigkeit;
- Fig. 2: eine zentrale Schnittansicht durch die Austragvorrichtung der Fig. 1, mit daran angeschlossener Spritze, beim Aufziehen einer Flüssigkeit;
- Fig. 3: eine Teil-Schnittansicht des in den Figuren 1 und 2 markierten Bereiches;
- Fig.4: eine perspektivische Ansicht des Kolbens der in der Fig. 1 gezeigten Austragvorrichtung;
- Fig. 5: eine Seitenansicht des Kolbens der in der Fig. 1 gezeigten Austragvorrichtung;
- Fig. 6: eine zentrale Schnittansicht durch die Austragvorrichtung der Fig. 1, mit daran angeschlossenem Vial, nach erfolgtem Aufziehen einer Flüssigkeit;
- Fig. 7: eine Teil-Schnittansicht des in der Figur 6 markierten Bereiches;
- Fig. 8: eine zentrale Schnittansicht durch die Austragvorrichtung der Fig. 1, mit daran angeschlossener Verschlusskappe, beim Vermischen des Produktes;
- Fig. 9: eine Teil-Schnittansicht des in der Figur 8 markierten Bereiches;
- Fig. 10: eine zentrale Schnittansicht durch die Austragvorrichtung der Fig. 1, mit daran angeschlossenem Injektionsaufsatz, beim Austragen des Produktes; sowie
- Fig. 11: eine Teil-Schnittansicht des in der Figur 10 markierten Bereiches.

### BESCHREIBUNG BEVORZUGTER AUSFÜHRUNGSFORMEN

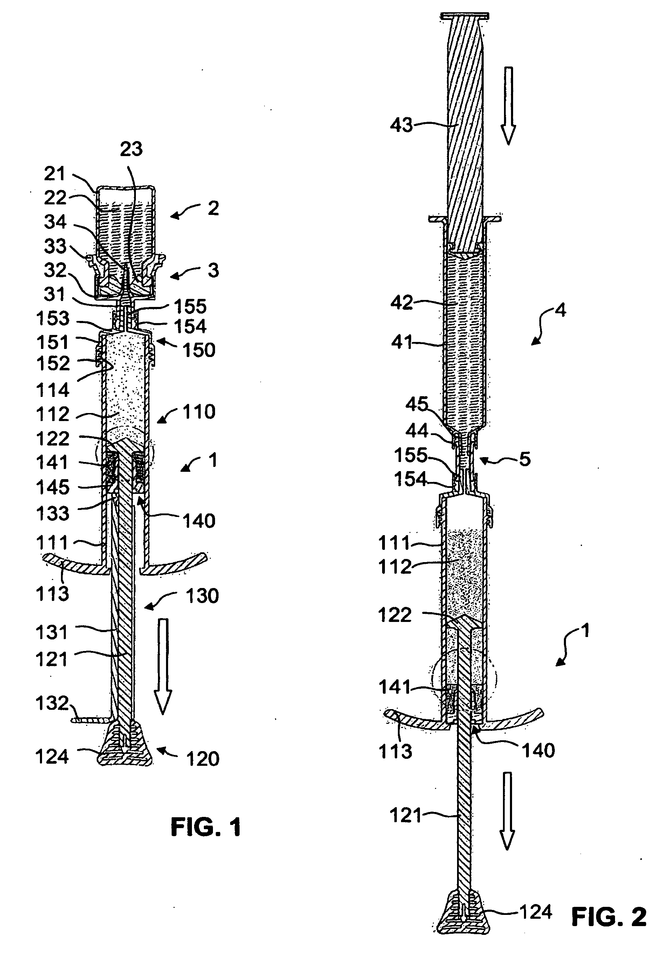

Die Figuren 1 bis 11 zeigen eine erfindungsgemässe Austragvorrichtung 1 gemäss einer bevorzugten Ausführungsform. Bei der Austragvorrichtung 1 dieser Ausführungsform handelt es sich um eine Spritze, welche beispielsweise für das Mischen und die Verabreichung eines Medikamentes oder zum Mischen und Austragen eines Klebstoffs oder eines Knochenzements verwendet wird. Die Austragvorrichtung 1 weist ein Gehäuse 110 auf, in welchem ein Vorschubelement 120 entlang einer axialen Längsrichtung verschiebbar angeordnet ist. Das Vorschubelement 120 ist dabei von einem proximalen Ende her in das Gehäuse 110 eingeschoben. Im Gehäuse 110 ist ausserdem ein Kolben 140 angeordnet, welcher relativ zum Gehäuse 110 und zum Vorschubelement 120 in Längsrichtung verschiebbar ist. Bei der vorliegenden Ausführungsform ist zudem ein Sicherungselement 130 am Vorschubelement 120 anbringbar und am distalen Ende ist ein Anschlussaufsatz 150 auf das Gehäuse 110 aufgeschraubt.

Das Gehäuse 110, welches auch als Spritzenkörper bezeichnet werden kann, weist eine umlaufende, einen Hohlzylinder bildende Seitenwand 111 auf, welche ein proximales und ein distales Ende hat. Die Seitenwand 111 begrenzt dabei ein sich in ihrem Innern erstreckendes, zylindrisches Reservoir 112. Durch seine zylindrische Form definiert das Reservoir 112 dabei eine axiale Längsrichtung und eine dazu radiale Richtung der Austragvorrichtung 1 sowie eine Reservoirmittelachse. Im Bereich des proximalen Endes der Seitenwand 111 sind an diametral gegenüberliegenden Seiten zwei radial nach aussen hin abstehende Halteflügel 113 angeordnet. Die Halteflügel 113 sind dabei leicht in die distale Richtung hin gebogen. Vom proximalen Gehäuseende her ist das Reservoir 112 durch eine proximale Öffnung des Gehäuses 110 zugänglich, wobei die proximale Gehäuseöffnung einen Innendurchmesser aufweist, welcher im Vergleich zu demjenigen der Seitenwand 111 kleiner ausgebildet ist. Im Bereich des distalen Gehäuseendes weist die Seitenwand 111 ein umlaufendes Aussengewinde 114 auf.

Der Anschlussaufsatz 150 weist eine Deckfläche 153 mit einem umlaufenden Mantel 151 auf. Auf der Innenseite des Mantels 151 ist ein Innengewinde 152 angebracht, welches komplementär zum Aussengewinde 114 des Gehäuses 110 ausgebildet ist. Auf der dem Mantel 151 gegenüberliegenden Seite weist die Deckfläche 153 einen männlichen Luer-Konus 155, welcher eine distale Austrittsöffnung des Gehäuses 110 umgibt, sowie eine dazu konzentrische Verriegelungshülse 154 auf. Die Verriegelungshülse 154, welche ein Innengewinde aufweist, bildet zusammen mit dem männlichen Luer-Konus 155 einen Anschluss der Austragvorrichtung 1, welcher zum Anschliessen von unterschiedlichen Anschlusselementen, wie z.B. Vials 2, anderen Spritzen, Injektionsnadeln 7 oder Anschlussadaptern 5 geeignet ist.

Das Vorschubelement 120 weist eine in Längsrichtung langgestreckte Kolbenstange 121 auf, an deren distalen Ende ein Mischelement 122 ausgebildet ist. Das Mischelement 122 erstreckt sich von der Kolbenstange 121 in die radiale Richtung. Dabei kann das Mischelement 122 auf unterschiedliche Art und Weise gemäss dem Stand der Technik ausgestaltet sein. Es kann beispielsweise einen äusseren, konzentrisch zur Kolbenstange 121 angeordneten Ring aufweisen, welcher über mehrere gerade oder gebogene Stege mit der Kolbenstange 121 verbunden ist. Das Mischelement 122 kann aber auch als eine Platte ausgebildet sein, welche mehrere Durchgangsöffnungen aufweist. Vorteilhaft weist das Mischelement 122 einen Aussendurchmesser auf, der nur geringfügig kleiner als der Innendurchmesser der Seitenwand 111 des Gehäuses 110 ist. Auf der proximalen Seite des Mischelementes 122 ist im Übergangsbereich zwischen dem Mischelement 122 und der Kolbenstange 121 ein umlaufender, sich von der Kolbenstange 121 aus nur geringfügig radial nach aussen hin erstreckender Absatz 125 ausgebildet, welcher eine Aussenkante aufweist. Am proximalen Ende der Kolbenstange 121 ist mittels einer Schnappverbindung ein Betätigungsgriff 124 angebracht.

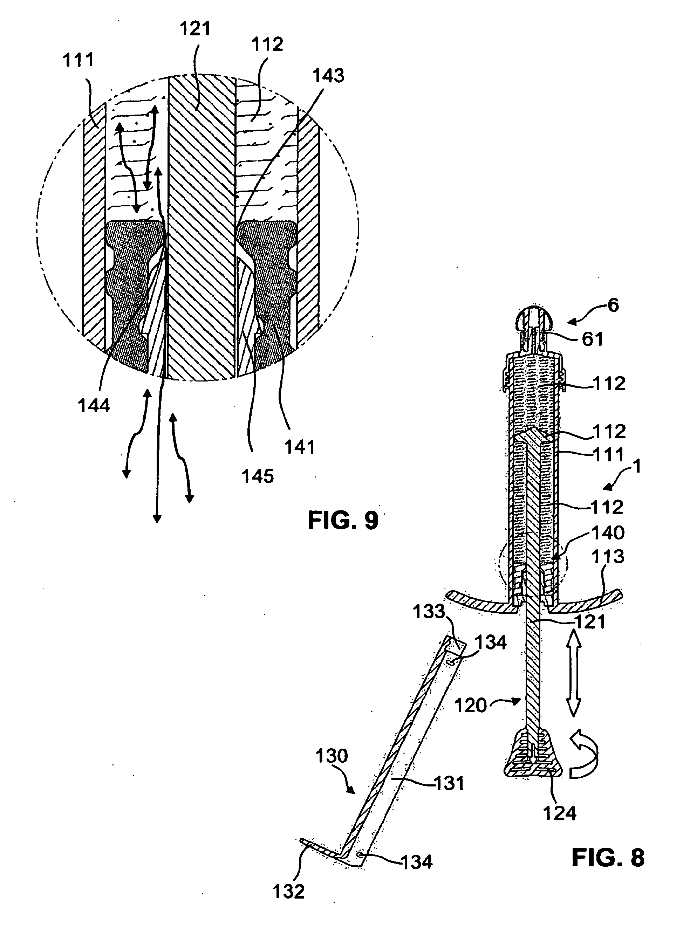

Das Sicherungselement 130, welches insbesondere in der Figur 8 gezeigt ist, weist im vorliegenden Ausführungsbeispiel einen Hauptabschnitt 131 auf, welcher einen bogenförmigen Querschnitt aufweist. Der Querschnitt des Hauptabschnittes 131 erstreckt sich dabei über einen Winkelbereich um die Längsachse, der geringfügig grösser als 180° ist. Auf seiner radialen Innenseite kann der Hauptabschnitt 131 Rastnasen 134 aufweisen. An seinem distalen Ende geht der Hauptabschnitt 131 in ein Anschlagelement 133 über. Am proximalen Ende des Hauptabschnittes 131 ist ein Greifflügel 132 angebracht, welcher rechtwinklig radial nach aussen hin wegragt.

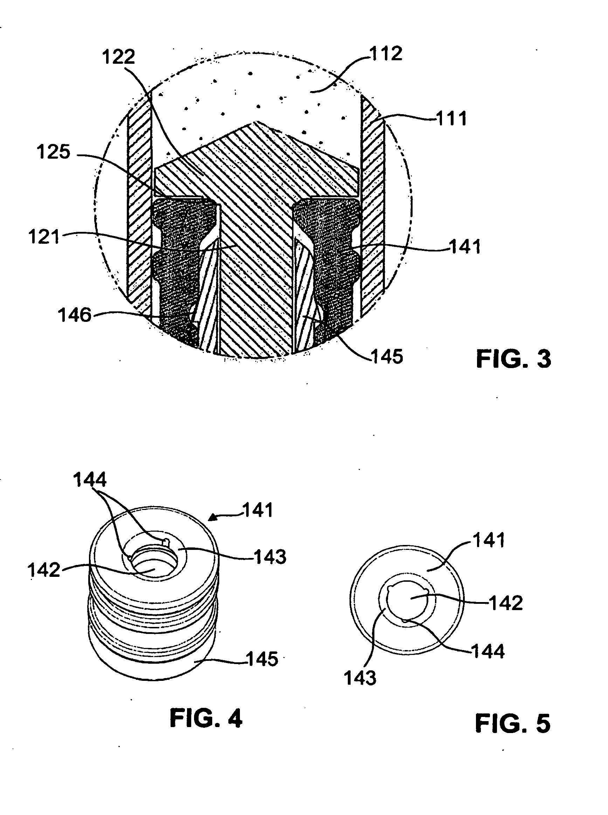

Der Kolben 140 der vorliegenden Ausführungsform ist insbesondere in den Figuren 4 und 5 gezeigt. In der vorliegenden Ausführungsform weist der Kolben 140 ein Dichtelement 141 sowie eine Trägerhülse 145 auf. Das Dichtelement 141 hat eine im Wesentlichen hohlzylindrische Form mit einer durchgehenden Öffnung. An der radialen Aussenseite des Dichtelementes 141 sind mehrere umlaufende Erhöhungen ausgebildet, welche dazwischen angeordnete Vertiefungen begrenzen. Auf der radialen Innenseite des Dichtelementes 141 ist eine umlaufende Rastrille ausgebildet. Im Bereich des distalen Endes des Dichtelementes 141 ist zudem eine umlaufende Dichtlippe ausgebildet, welche radial nach innen ragt, wodurch die durchgehende Öffnung des Dichtelementes 141 an dieser Stelle verengt ist. In die distale Richtung weitet sich diese Dichtlippe radial nach aussen hin auf und bildet dadurch einen Kontaktbereich 143. Die Dichtlippe weist mehrere, in der vorliegenden Ausführungsform genau drei, radiale Vertiefungen 144 auf, welche Entlüftungsöffnungen des Kolbens 140 bilden. Die Vertiefungen 144 sind hier in regelmässigen Abständen über den Umfang verteilt und radial nach innen hin offen ausgebildet.

Die Trägerhülse 145 bildet ein Halteelement, welches zum Halten des Dichtelements dient. Die Trägerhülse 145 weist einen sich in Längsrichtung erstreckenden Verbindungsbereich auf, welcher im Wesentlichen hohlzylinderförmig ausgebildet ist und eine axiale, durchgehende Öffnung 142 aufweist. Auf der radialen Aussenseite dieses Verbindungsbereiches sind mehrere Rastelemente 146 ausgebildet. Am proximalen Ende des Verbindungsbereiches ist ein umlaufender, radial nach aussen hin ragender Flansch ausgebildet. In einer alternativen Ausführungsform könnte die Trägerhülse 124 jedoch auch weggelassen werden. Der Kolben 140 könnte dann zum Beispiel nur aus einem Dichtelement bestehen.

In der vorliegenden Ausführungsform sind das Gehäuse 110, das Vorschubelement 120 (mit Ausnahme des Betätigungsgriffes 124), das Sicherungselement 130, die Trägerhülse 145, das Dichtelement 141 sowie der Anschlussaufsatz 150 jeweils einstückig im Spritzgussverfahren aus einem Kunststoff hergestellt. Das Dichtelement 141 ist dabei aus einem insbesondere elastischen und flexiblen Kunststoff hergestellt. Der Kolben 140 könnte in einer anderen Ausführungsform jedoch auch einstückig und zum Beispiel im Zweikomponenten-Spritzgussverfahren hergestellt sein. Auch könnte der Anschlussaufsatz 150 direkt einstückig mit dem Gehäuse 110 verbunden sein. Ebenso könnte der Betätigungsgriff 124 einstückig an der Kolbenstange 121 angebracht sein.

Das Zusammenwirken der verschiedenen Elemente sowie die Funktion der Austragvorrichtung 1 wird im Folgenden anhand der Figuren 1 bis 11 beschrieben.

Im Reservoir 112 der in Figur 1 gezeigten Austragvorrichtung 1 ist eine pulverförmige erste Substanz gelagert. In der in Figur 1 dargestellten Situation wird aus einem Vial 2 eine Flüssigkeit in die Austragvorrichtung 1 aufgezogen. In proximaler Richtung ist das Reservoir 112 dabei durch das Vorschubelement 120 und den Kolben 140 verschlossen, welche aneinander gekoppelt sind. Das Vorschubelement 120 ragt durch den Kolben 140 hindurch und liegt mit der proximalen Seite des Mischelementes 122 an diesem an. Das Dichtelement 141 ist auf die Trägerhülse 145 aufgesetzt, so dass diese von der proximalen Seite des Dichtelementes 141 her in dieses hineinragt. Die Rastelemente 146 der Trägerhülse 145 sind dabei in die auf der radialen Innenseite des Dichtelementes 141 ausgebildete Rastrille eingerastet. Auf seiner radialen Aussenseite liegt das Dichtelement 141 umlaufend und luftdicht an der Seitenwand 111 an. Der Absatz 125 des Mischelements 122 liegt zudem umlaufend und luftdicht am Kontaktbereich 143 des Kolbens 140 an. Er verschliesst zudem auch die radialen Vertiefungen 144 des Kolbens 140. Das Reservoir 112 ist dadurch in proximaler Richtung luftdicht verschlossen.

Das Sicherungselement 130 ist derart an der Kolbenstange 121 angebracht, dass es diese mit seinem U-förmigen Querschnitt umgibt. Dabei ist das Sicherungselement 130 zwischen dem Betätigungsgriff 124 und dem radial abragenden Flansch der Trägerhülse 145 eingeklemmt, wodurch das Sicherungselement 130 den Kolben 140 gegen das Mischelement 122 und insbesondere gegen den Absatz 125 hin drückt und somit den Durchgang zwischen dem Kontaktbereich 143 und dem Absatz 125 luftdicht abdichtet. Der Kolben 140 ist somit mit dem Vorschubelement 120 gekoppelt und mit Hilfe von diesem relativ zum Gehäuse 110 in Längsrichtung verschiebbar. Das Mischelement 122 bildet dann also einen distalen Anschlag für den Kolben 140. Das Dichtelement 141 wird dabei geringfügig in Längsrichtung komprimiert und dadurch in die radiale Richtung nach aussen zur Seitenwand 111 hin sowie nach innen zur Kolbenstange 121 hin gedrückt. Das Sicherungselement 130 liegt am proximalen Ende des Gehäuses 110 mit dem Hauptabschnitt 131 an der Innenkante der proximalen Gehäuseöffnung an, wodurch ein Entfernen des Sicherungselementes 130 von der Kolbenstange 121 in dieser Position verunmöglicht ist.

An den Lueranschluss 154, 155 des auf das Gehäuse 110 aufgeschraubten Anschlussaufsatzes 150 ist ein Anschlusselement 3 angeschlossen, in welchem ein Vial 2 gehalten ist. Das Anschlusselement 3 weist dazu einen weiblichen Luer-Konus 31 mit einem Aussengewinde sowie einen Aufnahmebereich 32 zur Aufnahme des Vials 2 auf. Ein Durchstechelement 34 des Anschlusselementes 3 dient dazu, ein Verschlusselement 23 des Vials 2 aufzustechen und eine Verbindung zu einem Reservoir 22, welches von einer Behälterwand 21 des Vials 2 begrenzt ist, herzustellen. Am Anschlusselement 3 angebrachte Lösegriffe 33 erleichtern ein Trennen des Vials 2 vom Anschlusselement 3.

Das Reservoir 22 des Vials 2 ist hier mit einer Flüssigkeit gefüllt, welche in das Reservoir 112 des Gehäuses 110 aufgezogen wird, indem das Vorschubelement 120 mit dem Kolben 140 in die proximale Richtung (Pfeilrichtung in Figur 1) aus dem Gehäuse 110 herausgezogen wird. Aufgrund des luftdichten Anliegens des Kolbens 140 an der Seitenwand 111 einerseits und am Vorschubelement 120 andererseits ergibt sich dabei ein Unterdruck im Reservoir 112, wodurch die im Reservoir 22 des Vials 2 gelagerte Flüssigkeit durch die vom Luer-Konus 155 umgebene Austrittsöffnung in das Reservoir 112 hineingezogen wird.

Die Flüssigkeit kann anstelle aus einem Vial 2 auch aus einer Spritze 4, wie in Figur 2 gezeigt ist, in das Reservoir 112 übertragen werden. Dazu kann der Lueranschluss 154, 155 der Austragvorrichtung 1 über einen Anschlussadapter 5 mit einem gleichartig ausgebildeten männlichen Luer-Konus 45 und einer Verriegelungshülse 44 der Spritze 4 verbunden werden. Eine derartige handelsübliche Spritze 4 weist ein Gehäuse 41 mit einem Reservoir 42 auf, in das eine Kolbeneinheit 43 einschiebbar ist, um in einer bekannten Art und Weise ein fluides Produkt aus dem Reservoir 42 auszustossen. Die Übertragung der in der Spritze 4 enthaltenen Flüssigkeit in das Reservoir 112 der Austragvorrichtung 1 kann grundsätzlich auf die selbe Art und Weise erfolgen, wie dies vorstehend für die Übertragung aus einem Vial beschrieben wurde, d.h., indem das Vorschubelement 120 mit daran angebrachtem Sicherungselement 130 zurückgezogen wird, wobei der Kolben 140 das Reservoir 112 luftdicht abschliesst und einen Unterdruck erzeugt. Im Beispiel der Fig. 2 erfolgt die Übertragung allerdings auf eine andere Art und Weise. Die Austragvorrichtung 1 wurde hier in einem Zustand ausgeliefert, in dem sich der Kolben 140 von Anfang an in einer proximalen Endposition innerhalb des Gehäuses 110 befindet. In dieser Stellung wurde das Sicherungselement 130 entfernt, und das Vorschubelement wurde durch den Kolben 140 hindurch so weit in die distale Richtung vorgeschoben, dass das Mischelement 122 nicht mehr am Dichtelement 141 anliegt. Hierdurch sind nun die als Entlüftungsöffnungen wirkenden radialen Vertiefungen 144 am Dichtelement 141 freigegeben, wie dies nachstehend noch näher im Zusammenhang mit der Fig. 9 erläutert wird. Die Flüssigkeit wird nun von der Spritze 4 in das Reservoir 112 übertragen, indem die Kolbeneinheit 43 der Spritze in die Spritze hineingestossen wird. Dabei verdrängt die in das Reservoir 112 gelangende Flüssigkeit die darin anfänglich vorhandene Luft und wird von der pulverförmigen Substanz absorbiert. Die Luft kann dagegen durch die Entlüftungsöffnungen entweichen. Dadurch wird eine Befüllung des Reservoirs 112 ermöglicht, ohne dass ein Überdruck entsteht.

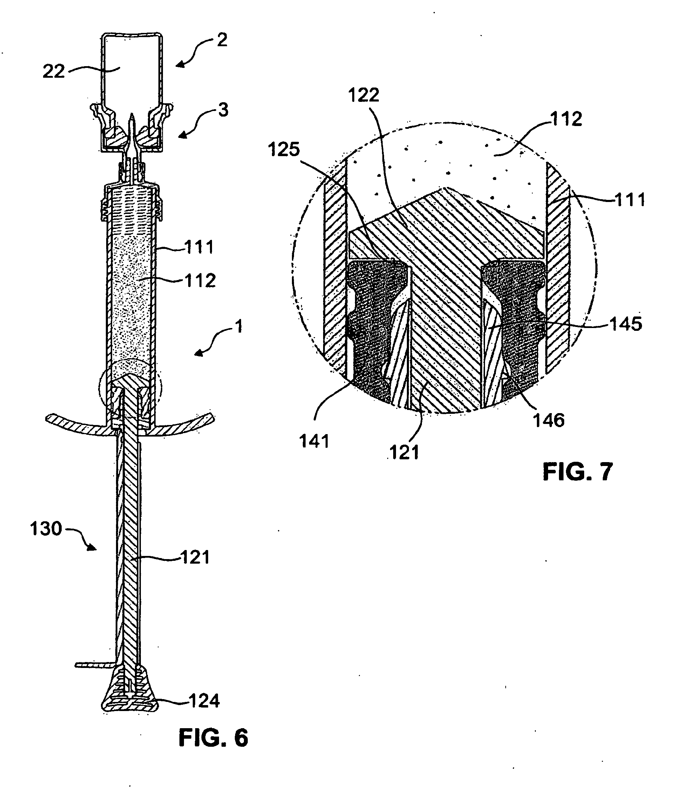

In den Figuren 6 und 7 ist die Situation gezeigt, in der eine Flüssigkeit vollständig aus einem Vial 2 in das Reservoir 112 der Austragvorrichtung 1 übertragen wurde. Das Vorschubelement 120 ist dabei soweit aus dem Gehäuse 110 herausgezogen, dass die Trägerhülse 145 des Kolbens 140 einen proximalen Anschlag im Bereich des proximalen Endes des Gehäuses 110 bildet. Die aufgezogene Flüssigkeit sowie die pulverförmige Substanz sind jedoch noch nicht vollständig miteinander vermischt, sondern bilden noch zwei Phasen. In der in Figur 6 gezeigten Position des Vorschubelementes 120 relativ zum Gehäuse 110 ist das Sicherungselement 130 von der Kolbenstange 121 entfernbar.

Nach Entfernen des Sicherungselementes 130 vom Vorschubelement 120 (Figuren 8 und 9) befindet sich der Kolben 140 in Bezug auf das Vorschubelement 120 in einem ungekoppelten Zustand. Das Vorschubelement 120 ist dann relativ zum Kolben 140 in Längsrichtung verschiebbar. Da insgesamt eine grössere Fläche des Dichtelementes 141 an der Seitenwand 111 als an der Kolbenstange 121 anliegt, bleibt der Kolben 140 bei einem Verschieben des Vorschubelementes 120 aufgrund der resultierenden Reibungskräfte relativ zum Gehäuse 110 unbeweglich. Das Vorschubelement 120 kann somit zum Vermischen der Flüssigkeit mit der pulverförmigen Substanz im Reservoir 112 beliebig vor- und rückwärts bewegt und um die Längsachse gedreht werden (Pfeilrichtungen in Fig. 8), ohne dabei den Kolben 140 aus seiner Lage im proximalen Bereich des Gehäuses 110 zu bewegen. Mit Hilfe des Mischelementes 122 sind die beiden Substanzen miteinander vermischbar. Die distale Austrittsöffnung des Austragvorrichtung 1 ist dabei vorteilhaft durch eine Verschlusskappe 6 verschlossen, um ein Austreten der zu vermischenden Substanzen zu verhindern. Die Verschlusskappe 6 weist dazu einen weiblichen Luer-Konus 61 mit Aussengewinde auf.

Dadurch, dass das Mischelement 122 und insbesondere der Absatz 125 im ungekoppelten Zustand nicht mehr am Dichtelement 141 anliegen, ist ein luftdurchlässiger Durchgang vom Reservoir 112 durch die radialen Vertiefungen 144 und durch den verbleibenden Raum zwischen der Kolbenstange 121 und der Durchgangsöffnung 142 der Trägerhülse 145 nach aussen hin freigegeben (siehe Pfeile in Fig. 9). Ein im Reservoir 112 aufgrund der Bewegungen des Mischelements 122 durch dessen Verdrängervolumen oder durch chemische Reaktionen entstehender Unter- oder Überdruck wird somit nach aussen hin ausgeglichen. Aufgrund der kleinen Dimensionierung der radialen Vertiefungen 144 ist das Reservoir 112 jedoch in Bezug auf pulver- und pastenförmige Substanzen nach aussen hin auch in diesem ungekoppelten Zustand weiterhin undurchlässig.

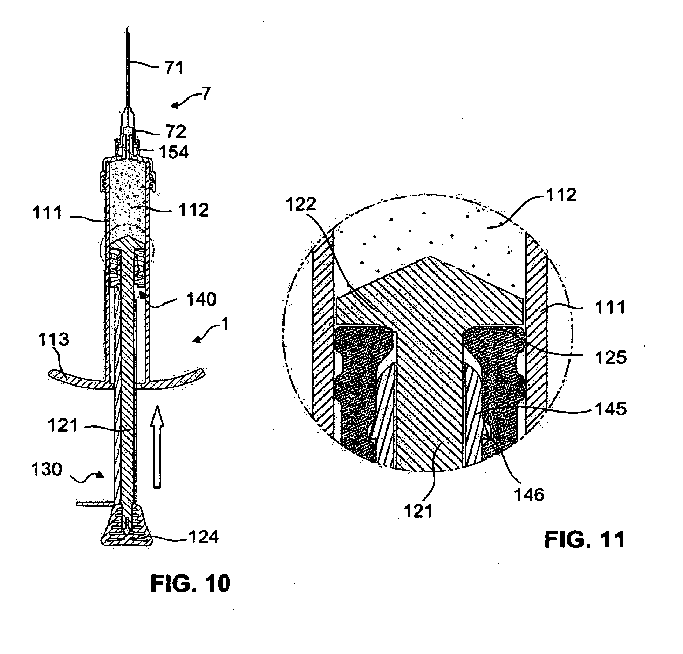

Zum Ausstossen des im Reservoir 112 enthaltenen, nun vermischten Produktes wird das Vorschubelement 120 dann bis zum proximalen Anschlag aus dem Gehäuse 110 zurückgezogen und das Sicherungselement 130 wiederum zwischen Betätigungsgriff 124 und Trägerhülse 145 eingeklemmt. Der Kolben 140 ist dann wieder mit dem Vorschubelement 120 gekoppelt und bildet einen luftdichten Abschluss des Reservoirs 112 in die proximale Richtung. Die Verschlusskappe 6 wird für das Ausstossen des vermischten Produktes abgenommen, und es kann beispielsweise ein Injektionsaufsatz 7 auf den Lueranschluss 154, 155 aufgesetzt werden. Der Injektionsaufsatz 7 kann dazu einen weiblichen Luer-Konus 72 aufweisen. Ausserdem kann am Injektionsaufsatz 7 eine Hohlnadel 71 vorgesehen sein, welche zur Injektion des in der Austragvorrichtung 1 enthaltenen Produktes in einen Patienten dient. Das Injizieren erfolgt durch Vorschieben des Vorschubelementes 120 in das Gehäuse 1 hinein, wie in den Figuren 10 (siehe Pfeilrichtung) und 11 gezeigt ist.

Die Erfindung ist selbstverständlich nicht auf das vorstehende Ausführungsbeispiel beschränkt, und eine Vielzahl von Abwandlungen ist möglich. So muss beispielsweise nicht zwingend ein Sicherungselement 130 vorgesehen sein. Das Vorschubelement könnte zum Beispiel auch radial nach aussen ragende Rastnasen aufweisen, welche in ähnlicher Weise wie bei einem Bajonettverschluss in komplementär dazu ausgebildete Vertiefungen der Trägerhülse mittels einer kombinierten Längs- und Rotationsbewegung einrastbar sind, um den Kolben mit dem Vorschubelement zu koppeln. Die Trägerhülse könnte dazu drehfest im Gehäuse geführt sein.

Die Austragvorrichtung könnte beispielsweise wie im Dokument US 7,736,049 auch ein zweites Reservoir aufweisen, das sich parallel zum ersten Reservoir erstreckt. Die Flüssigkeit könnte dann in diesem zweiten gelagert sein. Durch Hineinschieben eines zweiten Kolbens in dieses zweite Reservoir könnte die Flüssigkeit via einem Transferbereich in das erste Reservoir übertragen werden, wo sie dann mit einer darin enthaltenen Komponente vermischt und anschliessend ausgestossen würde. Der im ersten Reservoir vorgesehene erste Kolben wäre dann mit einem entsprechend ausgebildeten Vorschubelement, an dem ein Mischelement angebracht ist, koppel- und entkoppelbar.

Das Vorschubelement muss sich auch nicht zwingend durch den Kolben hindurch erstrecken. Denkbar ist auch eine Ausgestaltung der Austragvorrichtung, bei welcher die Kolbenstange des Vorschubelementes als ein Rohr ausgebildet ist, welches eine distale Öffnung mit einem Mischelement aufweist. Radial nach aussen hin könnte dieses Rohr beispielsweise mittels O-Ringen gegenüber der Gehäuseseitenwand abgedichtet sein. Der Kolben würde sich dann im Inneren dieses Rohres befinden, und beispielsweise an einer Betätigungsstange angebracht sein, welche mit dem Rohr koppelbar ist. Radiale Vertiefungen könnten dann, müssten aber nicht, auf der radialen Aussenseite des Kolbens ausgebildet sein.

Anstelle von radialen Vertiefungen auf der Innenseite bzw. Aussenseite des Dichtelementes könnten auch Entlüftungsöffnungen in Form von durchgehenden Löchern ausgebildet sein. Das Vorschubelement müsste dann entsprechend derart ausgebildet sein, dass es im gekoppelten Zustand diese Löcher bzw. Vertiefungen verschliesst.

Die durchgehenden Löcher oder radialen Vertiefungen am Dichtelement können grundsätzlich jedoch auch weggelassen werden. Das Dichtelement könnte auch einfach im gekoppelten Zustand mit einer distalen Stirnseite gegen einen umlaufenden, in die proximale Richtung weisenden Anschlag des Vorschubelementes 120 angepresst sein, und den Luftdurchlass im ungekoppelten Zustand dadurch gewährleisten, dass der Innendurchmesser des Dichtelementes einen im Vergleich zum Aussendurchmesser der Kolbenstange geringfügig grösseren Durchmesser aufweist.

**BEZUGSZEICHENLISTE**

| | | | |
|---|---|---|---|
| 1 | Austragvorrichtung | 153 | Deckfläche |
| | | 154 | Verriegelungshülse |
| 110 | Gehäuse | 155 | Männlicher Luer-Konus |
| 111 | Seitenwand | | |
| 112 | Reservoir | 2 | Vial |
| 113 | Halteflügel | 21 | Behälterwand |
| 114 | Aussengewinde | 22 | Reservoir |
| | | 23 | Verschlusselement |
| 120 | Vorschubelement | | |
| 121 | Kolbenstange | 3 | Anschlusselement |
| 122 | Mischelement | 31 | Weiblicher Luer-Konus |
| 124 | Betätigungsgriff | 32 | Aufnahmebereich |
| 125 | Absatz | 33 | Lösegriffe |
| | | 34 | Durchstechelement |
| 130 | Sicherungselement | | |
| 131 | Hauptabschnitt | 4 | Spritze |
| 132 | Greifflügel | 41 | Gehäuse |
| 133 | Anschlagelement | 42 | Reservoir |
| 134 | Rastnase | 43 | Kolbeneinheit |
| | | 44 | Verriegelungshülse |
| 140 | Kolben | 45 | Männlicher Luer-Konus |
| 141 | Dichtelement | | |
| 142 | Durchgangsöffnung | 5 | Anschlussadapter |
| 143 | Kontaktbereich | | |
| 144 | Entlüftungsöffnung | 6 | Verschlusskappe |
| 145 | Trägerhülse | 61 | Weiblicher Luer-Konus |
| 146 | Rastelement | | |
| | | 7 | Injektionsaufsatz |
| 150 | Anschlussaufsatz | 71 | Hohlnadel |
| 151 | Mantel | 72 | Weiblicher Luer-Konus |
| 152 | Innengewinde | | |

## Patentansprüche

1. Austragvorrichtung (1) zum Mischen und Austragen eines Produktes, aufweisend
ein Gehäuse (110), welches ein Reservoir (112) für das Produkt begrenzt, mit einer umlaufenden Seitenwand (111) einem offenen proximalen Gehäuseende (113)
und einem distalen Gehäuseende (153) mit einer Austrittsöffnung (155),
ein Vorschubelement (120), welches vom proximalen Gehäuseende (113) her in das Gehäuse (110) hineinragt und entlang einer Längsrichtung relativ zum Gehäuse (110) verschiebbar ist, wobei das Vorschubelement (120) einen distalen Bereich mit einem Mischelement (122) zum Vermischen des Produktes aufweist,
und
einen Kolben (140), welcher proximal in Bezug auf das Mischelement (122) angeordnet ist, und welcher relativ zum Gehäuse (110) in Längsrichtung verschiebbar ist, um das Produkt durch die Austrittsöffnung (155) hindurch aus dem Reservoir (112) auszustossen,
wobei der Kolben (140) einen ungekoppelten Zustand aufweist, in welchem das Vorschubelement (120) relativ zum Kolben (140) in Längsrichtung verschiebbar ist, sowie einen mit dem Vorschubelement (120) gekoppelten Zustand aufweist, in welchem der Kolben (140) mittels des Vorschubelements (120) in Längsrichtung relativ zum Gehäuse (110) verschiebbar ist,
**dadurch gekennzeichnet, dass**
der Kolben (140) im gekoppelten Zustand zusammen mit dem Vorschubelement (120) das Reservoir (112) in proximaler Richtung luftdicht nach aussen hin abdichtet, und dass der Kolben (140) und/oder das Vorschubelement (120) im ungekoppelten Zustand mindestens eine Entlüftungsöffnung (144) in proximaler Richtung nach aussen hin freigeben.

2. Austragvorrichtung gemäss Anspruch 1, wobei sich das Vorschubelement (120) durch den Kolben (140) hindurch erstreckt.

3. Austragvorrichtung gemäss einem der Ansprüche 1 oder 2, wobei die Entlüftungsöffnung am Kolben (140) ausgebildet ist, und wobei das
Vorschubelement (120) die Entlüftungsöffnung (144) im gekoppelten Zustand luftdicht verschliesst.

4. Austragvorrichtung gemäss Anspruch 3, wobei die Entlüftungsöffnung (144) im gekoppelten Zustand durch das Mischelement (122) verschlossen ist.

5. Austragvorrichtung gemäss einem der Ansprüche 3 oder 4, wobei die Entlüftungsöffnung (144) als eine radiale Vertiefung ausgebildet ist.

6. Austragvorrichtung gemäss Anspruch 5, wobei die Entlüftungsöffnung (144) an einer radialen Innenseite des Kolbens (140) ausgebildet ist.

7. Austragvorrichtung gemäss Anspruch 6, wobei die radiale Innenseite des Kolbens (140) im Bereich der Entlüftungsöffnung (144) einen radial umlaufenden Kontaktbereich (143) aufweist, welcher sich in distaler Richtung radial nach aussen hin aufweitet, und wobei das Vorschubelement (120) einen radial umlaufenden Absatz (125) mit einer Aussenkante aufweist, welche im gekoppelten Zustand umlaufend luftdicht am Kontaktbereich (143) anliegt.

8. Austragvorrichtung gemäss einem der vorhergehenden Ansprüche, wobei der Kolben (140) und das Vorschubelement (120) das Reservoir (112) sowohl im gekoppelten Zustand als auch im ungekoppelten Zustand derart abdichten, dass das Reservoir in die proximale Richtung in Bezug auf pulver- und pastenförmige Substanzen nach aussen hin undurchlässig ist.

9. Austragvorrichtung gemäss einem der vorhergehenden Ansprüche, wobei der Kolben (140) eine Trägerhülse (145) sowie ein an der Trägerhülse (145) angebrachtes, die Trägerhülse (145) umgebendes Dichtelement (141) aufweist.

10. Austragvorrichtung gemäss Anspruch 9, wobei die Entlüftungsöffnung (144) am Dichtelement (141) ausgebildet ist.

11. Austragvorrichtung gemäss einem der vorhergehenden Ansprüche, wobei der Kolben (140) nur dann vom Vorschubelement (120) entkoppelbar ist, wenn der Kolben (140) bis zu einem proximalen Anschlag aus dem Gehäuse (110) herausgezogen ist.

12. Austragvorrichtung gemäss einem der vorhergehenden Ansprüche, ausserdem aufweisend ein Sicherungselement (130), welches derart in Längsrichtung verschiebefest an das Vorschubelement (120) anbringbar ist, dass der Kolben (140) in Längsrichtung zwischen dem Sicherungselement (130) und dem Vorschubelement (120) fixiert ist und dadurch mit dem Vorschubelement (120) gekoppelt ist.

13. Austragvorrichtung gemäss Anspruch 12, wobei das Mischelement (122) im gekoppelten Zustand einen distalen Anschlag für den Kolben (140) bildet.

14. Austragvorrichtung gemäss einem der Ansprüche 12 oder 13, wobei das Sicherungselement (130) im gekoppelten Zustand in proximaler Richtung einen Anschlag mit einem am Vorschubelement (120) angebrachten Betätigungsgriff (124) bildet und in distaler Richtung einen Anschlag mit dem Kolben bildet.

## Claims

1. A discharging device (1) for mixing and discharging a product, comprising:
a housing (110), which delimits a reservoir (112) for the product, with a circumferential sidewall (111), an open proximal housing end and a distal housing end (153) with an outlet opening (155);
an advancement element (120), which projects into the housing (110) from the proximal housing end (113) and which can be slid along a longitudinal direction relative to the housing (110), wherein the advancement element (120) comprises a distal region with a mixing element (122) for mixing the product; and
a piston (140), which is proximally arranged relative to the mixing element (122) and which can be slid relative to the housing (110) in the longitudinal direction in order to eject the product from the reservoir (112) through the outlet opening (155),
wherein the piston (140) has an uncoupled state in which the advancement element (120) can be slid relative to the piston (140) in the longitudinal direction, and has a state in which it is coupled to the advancement element (120), in which state the piston (140) can be slid in the longitudinal direction relative to the housing (110) by means of the advancement element (120), **characterised in that**
the piston (140) in the coupled state together with the advancement element (120) seals the reservoir (112) in the proximal direction in an airtight manner towards the outside, and **in that** the piston (140) and/or the advancement element (120) in the uncoupled state frees at least one venting opening (144) in proximal direction towards the outside.

2. The discharging device according to claim 1, wherein the advancement element (120) extends through the piston (140).

3. The discharging device according to one of claims 1 or 2, wherein the venting opening is arranged on the piston (140), and wherein in the coupled state the advancement element (120) closes off the venting opening (144) in an airtight manner.

4. The discharging device according to claim 3, wherein in the coupled state the venting opening (144) is closed off by the mixing element (122).

5. The discharging device according to one of claims 3 or 4, wherein the venting opening (144) is designed as a radial indentation.

6. The discharging device according to claim 5, wherein the venting opening (144) is arranged on a radial inside of the piston (140).

7. The discharging device according to claim 6, wherein the radial inside of the piston (140) comprises a radially circumferential contact region (143) in the region of the venting opening (144), which contact region (143) widens in the distal direction radially towards the outside, and wherein the advancement element (120) comprises a radially circumferential recess (125) with an outside edge, which outside edge in the coupled state rests against the contact region (143) so as to provide a circumferential seal.

8. The discharging device according to any one of the preceding claims, wherein the piston (140) and the advancement element (120) seal the reservoir (112) both in the coupled state and in the uncoupled state in such a manner that the reservoir in the proximal direction is impermeable towards the outside for powdery and paste-like substances.

9. The discharging device according to any one of the preceding claims, wherein the piston (140) comprises a carrier sleeve (145) and a sealing element (141), affixed to the carrier sleeve (145), which sealing element (141) surrounds the carrier sleeve (145).

10. The discharging device according to claim 9, wherein the venting opening (144) is arranged on the sealing element (141).

11. The discharging device according to any one of the preceding claims, wherein the piston (140) can be decoupled from the advancement element (120) only when the piston (140) has been withdrawn from the housing (110) up to a proximal end stop.

12. The discharging device according to any one of the preceding claims, further comprising a securing element (130) that can be attached to the advancement element (120) so that the securing element (130) is non-displaceable in the longitudinal direction, so that the piston (140) is fixed in the longitudinal direction between the securing element (130) and the advancement element (120), thus being coupled to the advancement element (120).

13. The discharging device according to claim 12, wherein in the coupled state the mixing element (122) provides a distal end stop for the piston (140).

14. The discharging device according to one of claims 12 or 13, wherein in the coupled state the securing element (130) in the proximal direction forms an end stop with an actuating handle (124) that is affixed to the advancement element (120), and in the distal direction forms an end stop with the piston.

## Revendications

1. Dispositif de déversement (1) pour le mélange et le déversement d'un produit, présentant
un boitier (110) qui délimite un réservoir (112) pour le produit, comprenant une paroi latérale périphérique (111), une extrémité de boîtier (113) proximale ouverte et une extrémité de boitier distale (153) comprenant une ouverture de sortie (155),
un élément d'avancement (120) qui fait saillie de l'extrémité de boîtier proximale (113) dans le boitier (110) et peut se déplacer par rapport au boitier (110) le long d'un sens longitudinal, sachant que l'élément d'avancement (120) présente une partie distale avec un élément mélangeur (122) pour mélanger le produit, et un piston (140) qui est disposé de façon proximale par rapport à l'élément mélangeur (122) et qui peut se déplacer dans le sens longitudinal par rapport au boitier (110), pour éjecter le produit hors du réservoir (112) à travers l'ouverture de sortie (155),
sachant que le piston (140) présente un état non couplé dans lequel l'élément d'avancement (120) peut être déplacé dans le sens longitudinal par rapport au piston (140), ainsi qu'un état couplé à l'élément d'avancement (120) dans lequel le piston (140) peut être déplacé via l'élément d'avancement (120) dans le sens longitudinal par rapport au boitier (110),
**caractérisé en ce que**
le piston (140), dans l'état couplé, conjointement avec l'élément d'avancement (120), étanchéifie le réservoir (112) contre l'air vers l'extérieur dans le sens proximal, et que le piston (140) et/ou l'élément d'avancement (120), dans l'état non couplé, libère (nt) vers l'extérieur au moins une ouverture de ventilation (144) dans le sens proximal.

2. Dispositif de déversement selon la revendication 1, dans lequel l'élément d'avancement (120) s'étend à travers le piston (140).

3. Dispositif de déversement selon la revendication 1 ou 2, dans lequel l'ouverture de ventilation est formée sur le piston (140), et sachant que l'élément d'avancement (120) ferme l'ouverture de ventilation (144) en étanchéité à l'air dans l'état couplé.

4. Dispositif de déversement selon la revendication 3, dans lequel l'ouverture de ventilation (144) est fermée par l'élément mélangeur (122) dans l'état couplé.

5. Dispositif de déversement selon l'une des revendications 3 ou 4, dans lequel l'ouverture de ventilation (144) est formée en tant que creux radial.

6. Dispositif de déversement selon la revendication 5, dans lequel l'ouverture de ventilation (144) est formée sur un côté intérieur radial du piston (140).

7. Dispositif de déversement selon la revendication 6,
dans lequel le côté intérieur radial du piston (140) présente au niveau de l'ouverture de ventilation (144), une zone de contact périphérique radiale (143), qui s'élargit radialement vers l'extérieur dans le sens distal, et sachant que l'élément d'avancement (120) présente un épaulement radial périphérique (125) avec une arête extérieure, laquelle repose en étanchéité à l'air sur le pourtour de la zone de contact (143) dans l'état couplé.

8. Dispositif de déversement selon l'une des revendications précédentes, dans lequel le piston (140) et l'élément d'avancement (120) étanchéifient le réservoir (112) tant dans l'état couplé que dans l'état découplé, de telle façon que le réservoir ne laisse pas passer les substances pulvérulentes ou pâteuses vers l'extérieur dans le sens proximal.

9. Dispositif de déversement selon l'une des revendications précédentes, dans lequel le piston (140) présente un manchon porteur (145) ainsi qu'un élément d'étanchéité (141) placé sur le manchon porteur (145), entourant le manchon porteur (145).

10. Dispositif de déversement selon la revendication 9, dans lequel l'ouverture de ventilation (144) est formée sur l'élément d'étanchéité (141).

11. Dispositif de déversement selon l'une des revendications précédentes, dans lequel le piston (140) ne peut être découplé de l'élément d'avancement (120) que lorsque le piston (140) est tiré hors du boitier (110) jusqu'à une butée proximale.

12. Dispositif de déversement selon l'une des revendications précédentes, présentant en outre un élément de fixation (130) qui peut être placé en résistance au déplacement sur l'élément d'avancement (120) dans le sens longitudinal de telle sorte que le piston (140) est fixé entre l'élément de fixation (130) et l'élément d'avancement (120) dans le sens longitudinal et ainsi couplé à l'élément d'avancement (120).

13. Dispositif de déversement selon la revendication 12, dans lequel l'élément mélangeur (122) forme une butée distale pour le piston (140) dans l'état couplé.

14. Dispositif de déversement selon l'une des revendications 12 ou 13, dans lequel l'élément de fixation (130) forme, dans l'état couplé et dans le sens proximal, une butée avec une poignée d'actionnement (124) placée sur l'élément d'avancement (120) et forme une butée avec le piston dans le sens radial.
